# EUROPEAN PATENT APPLICATION

(11) **EP 3 241 555 A1**
(43) Date of publication of application: **08.11.2017**
(21) Application number: 15875292.3
(22) Date of filing: 29.12.2015
(51) Int. Cl.: A61K 31/662, A61P 31/20

(54) **USE OF PYROPHOSPHATE ION ANALOGUES FOR THE TREATMENT OF HPV INFECTION**

(30) Priority: 29.12.2014 ES 201431946
(71) Applicant: Agencia Pública Empresarial Sanitaria Costa Del Sol, 29603 Marbella (ES)
(72) Inventor: DEL BOZ GONZÁLEZ, Francisco Javier, 29603 Marbella (Málaga) (ES); HERNÁNDEZ IBAÑEZ, Carlos, 29603 Marbella (Málaga) (ES); ESCUDERO SANTOS, Isabel María, 29603 Marbella (Málaga) (ES)
(74) Representative: Hoffmann Eitle
(86) International application number: PCT/ES2015/070962
(87) International publication number: WO 2016/107955

(57) **Abstract**

The invention relates to the use of pyrophosphate ion analogues in the production of a drug for the prevention, alleviation or treatment of an HPV infection-related disease. The invention also relates to the pharmaceutical composition, patch or dressing, kit or device and uses.

## Description

### FIELD OF THE INVENTION

The present invention is comprised in the field of medicine and pharmacy, and relates to the use of pyrophosphate ion analogues, and more specifically phosphonoformic acid or foscarnet, in the production of a drug for the prevention, alleviation or treatment of an HPV infection-related disease.

### BACKGROUND OF THE INVENTION

In medicine, the term "papilloma" is used to generally refer to a benign epithelial tumor that grows exophytically, where it refers to lesions that originate in the skin, conjunctiva, mucous membranes or glandular ducts and grow thereon. Some authors advocate that it is perhaps more appropriate to restrict the use of this term to exophytic lesions of the genital and/or oral mucosa.

Human papillomaviruses (HPVs), the name from which the widespread term "papilloma" is derived, are a diverse group of DNA viruses from the *Papillomaviridae* family. Like all viruses from this family, HPVs only establish productive infections in the stratified epithelium of the skin and mucosae of humans and a variety of animals (ICD-10 B 97.7, b 95; ICD-9 079.4 078.1; 079; DiseasesDB 6032; MeSH D030361).

Around 200 different HPV types, most of which do not cause any sign/symptom in most people, have been identified. Some HPV types can cause warts (condylomata, if they originate in the genital area) while others can cause subclinical infections which (in a minority of cases) can result in cervical, vulvar, vaginal and anal cancer in women, and anal and penile cancer in men.

In medical terminology, a wart is an exophytic or non-exophytic skin/mucosal lesion caused by the human papillomavirus (HPV), although among laymen (and even among many healthcare workers) the term "wart" is an equivalent to the term "papilloma." Viral warts (or simply warts) are highly contagious, benign epidermal neoformations of viral origin. Warts are among the 10 most common dermatoses affecting children and adults of both sexes, with a prevalence of HPV infection of 79% in the general population (Lizárraga et al., 2009. Cent Dermatol Pascua, 18 (1): 5-18). They take on different forms and are generally exophytic, and can affect different areas of the skin and/or mucosae where their development is enhanced in specific population groups such as patients suffering from atopic dermatitis and patients whose immune systems are compromised for various reasons.

Depending on the serotype of the virus, the most commonly affected area is different. The three most common clinical forms are: vulgar warts (70%), plantar warts (26%) and flat warts (4%) (Woolf et al., 2005. Fitzpatrick's, Color Atlas & Synopsis of Clinical Dermatology. 5th Ed. Philadelphia; McGraw-Hill, 776-81; Arenas, 2005. Dermatología. Diagnóstico y tratamiento. 3rd Ed. Mexico; McGraw-Hill: 656-667).

**Table 1. HPV-induced diseases**

| **Disease** | **More commonly associated HPV types** |
|---|---|
| Vulgar wart | 2, 7 |
| Plantar wart | 1, 2, 4 |
| Flat warts | 3, 10 |
| Anogenital warts | 6, 11, 42, 43, 44, 55 and others |
| Genital epithelial neoplasias | 16, 18, 31, 33, 35, 39, 45, 51 |
| Epidermodysplasia verruciformis | More than 15 types |
| Focal epithelial hyperplasia (oral) | 13, 32 |
| Oral papillomas | 6, 7, 11, 16, 32 |
| Bowenoid papulosis | 16,18,33 |

Vulgar warts are fundamentally associated with HPV genotypes 1, 2, 4 and 7, and occur as localized outgrowths of the same color as the skin, with a keratotic surface that is rough to the touch and may appear cauliflower-like. Blackish brown spots corresponding to dermal papilla blood vessels in which clots are formed due to the presence of the virus are usually present on the surface thereof. They can appear on any skin surface, although preferably on the back of the hands and fingers, usually followed by the face, neck and scalp.

Flat warts are particularly associated with HPV genotype 3 and occur as slightly raised lesions that are 2-4 mm in diameter with a flat surface (as its name indicates) and yellowish or brownish color and are hard to distinguish from normal skin. They have a soft consistency and their surface is smooth to touch. They appear most frequently on the face and the back of the hands.

Plantar warts which, as its name indicates, appear on the soles of the feet, are also commonly referred to as corns or calluses. Plantar warts (*verruca pedis*; VP; Viral infections characterized by lesions on the skin and mucous membranes ICD-10: B07//ICD-9-CM Diagnosis Code 078.19) affect between 7 and 10% of the general population (Chicharro and Alonso, 2007), no significant differences were found between sexes with respect to the prevalence of this disease (Pique *et al.,* 1997; Lipke, 2006) and this lesion occurs most often in patients comprised between 5 and 20 years of age (Palomo, 2000. Podoscopio, 1 (11):24-32) where it has been calculated that 10% of adolescents suffer from said lesion. Adults affected with this virus can have a compromised immune system (Llorente, 2003. Podoscopia, 22: 4-12). The incidence therefore depends, in particular, on the age of the patients and their immune status, and can be made worse by excessive sweating. Furthermore, patients who have had plantar and palmer warts have possibilities of developing new lesions 3 times higher than unaffected patients (Palomo, 2000. Podoscopio, 1 (11): 24-32; Lipke, 2006. Clinical Medicine & Research, 4(4), 273-293). Plantar warts are usually small lesions, and due to the pressure the body applies on the soles of the feet, the skin over the wart may harden. They may or may not be painful, according to their size, progression time, location and human papillomavirus subtype. They sometimes cluster together giving rise to thickened plaques with a keratotic surface that are called mosaic warts. VPs are often differentiated from corns, on one hand, as blackish spots are observed on the surface thereof, and on the other, as the legs, like the hands, are found to be covered with these marks commonly referred to as fingerprints upon analyzing the lines of the skin. In the case of plantar warts, these lines surround the lesion, whereas in lesions other than VPs, cellular DNA is unchanged and the lines run through the lesion. Furthermore, VPs tend to cause pain when pressure is applied on the sides of the lesion rather than directly on it, whereas the opposite is true for corns where they cause pain when pressure is applied directly on them and not on their sides. The most common areas in which VPs occur are under the balls of the feet, on the side of the first toe and heel. They are usually transmitted in public swimming pools and toilets (when one goes barefoot), by sharing shoes, etc.

Anogenital warts (condylomata) have variable oncogenic potential and characteristics. Between 30 and 40 HPVs are typically transmitted by sexual contact, infecting the anogenital region. Some sexually transmitted HPV types (such as HPV types 6 and 11) can cause genital warts, although other HPV types which can infect the genitals do not cause any considerable signs of infection.

Furthermore, a persistent infection with a subgroup of about 13 sexually transmitted HPVs, referred to as «high-risk» HPVs, including types 16, 18, 31, 33, 35, 39, 45, 51, 52, 56, 58, 59, 68 -which are different from those causing warts- can enhance the development of:
- CIN (cervical intraepithelial neoplasia),
- VIN (vulvar intraepithelial neoplasia),
- PIN (penile intraepithelial neoplasia), or
- AIN (anal intraepithelial neoplasia).

These are precancerous lesions and can progress into invasive cancer. An HPV infection is a necessary factor in the development of almost all cervical cancer cases.

Several HPV types, particularly type 16, have been found to be associated with squamous cell oropharyngeal carcinoma, a form of head and neck cancer.

In fact, there is evidence that a large number of cancers originate from HPV infection (Zur Hausen 1996. Biochim Biophys Acta Oct 9; 1288(2):F55-F78) and these cancers include, among others: cervical cancer (cancer of the cervix uteri) and cancer in different organs such as the oral mucosa, tonsil, pharynx, larynx, esophagus, breast, prostate, ovary, urethra, and even skin cancer. Current research being done on melanocytic tumors imparts a relevant role to this virus, particularly in the aspects of progression of the disease (Dreau et al., 2000. Annals of Surgery 231:664-671). HPV-induced cancers often have viral sequences integrated in the cellular DNA. Some of the "early" HPV genes, such as E6 and E7, are known to act as oncogenes promoting tumor growth and malignant transformation. The p53 protein prevents cell growth in the presence of damaged DNA primarily through the BCL-2-associated X protein domain (BAX) (blocking the anti-apoptosis effects of the mitochondrial receptor BCL-2). Furthermore, p53 also over-regulates the p21 protein, blocking the formation of the cyclin D/Cdk4 complex, and thereby preventing RB phosphorylation and, in turn, impeding cell cycle progression by preventing E2F activation. In short, p53 is a tumor suppressor gene that stops the cell cycle when there is damaged DNA. E6 and E7 proteins work by inhibiting tumor suppressor genes; in a process of this type, E6 inhibits p53, while E7 inhibits p53, p21, and an RB.

A history of infection with one or more high-risk HPV types is considered to be a prerequisite for the development of cervical cancer; according to the "American Cancer Society, ACS", women without any history of the virus do not develop that type of cancer. Most HPV infections are quickly taken care of by the immune system and do not progress to cervical cancer. Due to the fact that the process of transforming normal cervical cells into cancerous cells is slow, cervical cancer occurs in women who have been infected with HPVs for a long time, usually a decade or more.

Sexually transmitted HPVs can also cause most anal cancers and about 25% of mouth and throat (oropharyngeal) cancers. The latter usually occurs in the area of the tonsils, and HPV is linked to an increase in oral cancer in non-smokers.

HPV types 6 and 11 can cause a rare condition known as recurrent respiratory papillomatosis (a respiratory papillomatosis), in which warts are formed in the larynx or in other areas of the respiratory tract. Those warts can recur frequently, require repeated surgical interventions, interfere with respiration, and in extremely rare cases, progress to cancer.

With respect to treatments, there are antiviral drugs that are specific for HPV (Gilaberte and Puig, 2000. Farmacia Profesional, 14 (12): 76-81), and the different treatments available are generally aimed at destroying all virus-infected cells, or causing the actual immune system of the individual to do the same.

It is not easy to remove warts by surgery as they have their own blood supply system which causes excessive bleeding and they can even regenerate more virulently. Additionally, it can compromise nerve endings, so removal or manipulation thereof may be very painful.

There are treatments based on the application of formulas with acidic and other keratolytic substances for eliminating virus-infected cells, i.e., the wart tissue, in several applications, for example, solutions containing 20-40% salicylic acid, master formulas containing 10% glutaraldehyde gel or 5-10% aqueous glutaraldehyde solution, topical retinoids such as 0.025 - 0.05% tretinoin for the treatment of flat warts, silver nitrate, etc.

Treatments available today include, among many others, destructive techniques (cryotherapy, ablative lasers such as CO₂ or erbium laser, electrocoagulation, surgical excision), light sources (pulsed dye laser, photodynamic therapy), immunostimulation- and immunomodulation-based treatments (dinitrochlorobenzene, squaric acid, interferon, imiquimod, cantharidin), antimitotic drugs (bleomycin, 5-fluorouracil, podophyllotoxin), intralesional immunotherapy (HPV and triple viral vaccines) and even systemic treatments with retinoids, and cimetidine. (Becelieri R, Johnson SM. Cutaneous warts: an evidence-based approach to therapy).

The last Cochrane review of 2014 (Kwok C, Gibbs S, Bennett C, Holland R, Abbott R. Topical treatments for skin warts. Cochrane Database of Systematic Reviews 2014 Issue 2. Art. No.: CD001781. DOI: 10.1002/14651858.CD001781) establishes salicylic acid- and aggressive cryotherapy-based therapies- as the only consistent evidence for the treatment of warts, where tests are still the most consistent for salicylic acid, although they only show a moderate therapeutic effect.

Chicharro and Alonso (2007. Revista Española de Podología, 18: 218-22) recommend starting from the simplest, least painful and least costly forms of treatment; in this sense, the first line of action would be performed on keratolytics where salicylic acid (20 - 40%) stands out due to its efficacy and ease-of-application with a cure rate between 60% and 80% (Gibbs and Harvey, 2003. Cochrane Database Syst Rev. (3):CD00178); Llorente et al., 2003. Podoscopia, 22: 4-12; Llarden et al., 2006. Formación Médica Continuada en Atención Primaria 13, 45-54).

The second line of action involves cryotherapy (application of liquid nitrogen at -196°C) whereby up to 80% of myrmecia-type plantar warts and 50% of mosaic-type warts are eliminated. The third line of action will include, among others, cytotoxic drugs (intralesional and podophyllin), the immunomodulators (iniquimod, cimetidine), 5-fluorouracil (alone or in combination with salicylic acid), topical immunotherapy with topical sensitizing agents such as dinitrochlorobenzene, topical zinc sulfate, interferon, intralesional candida antigen, photodynamic therapy and surgical excision or by means of laser. These treatments are often used on recalcitrant warts when the first and second line of action have failed (Micali et al., 2004. American Journal of Clinical Dermatology, 5: 311-317; Dall'Oglio F, D'Amico V, Nasca M, Micali G. 2012. Am. J. Clin. Dermatol. 13:73-96). Recurrences are extremely common with most treatments since they do not usually eliminate the virus entirely (Gibbs et al., 2003. Cochrane Database Syst Rev. (3):CD00178).

In recent years different publications, among which there is a need to highlight publications made by the authors of the present invention (Padilla L, del Boz J, Fernández Morano M, Arenas-Villafranca J, de Troya M. Successful treatment of periungual warts with topical cidofovir. Dermatol. Ther. 2014; 27:337-42; Fernández Morano T, del Boz J, González Carrascosa M, Tortajada B, de Troya M. Topical cidofovir for viral warts in children. J Eur Acad Dermatol Venereol.2011; 25:1480-90*;* Padilla España L, del Boz J, Fernández Morano T, Arenas Villafranca J, de Troya Martín M. Topical Cidofovir for plantar warts. Dermatol Ther. 2014; 27:89-93), have demonstrated the usefulness of an antiviral, i.e., cidofovir, in the treatment of HPV infections. Cidofovir is a cytidine analogue which inhibits the action of the DNA polymerase of different viruses, and although it is indicated and approved for the treatment (by means of intravenous infiltration) of cytomegalovirus chorioretinitis in HIV-infected patients, it must be used topically (by means of a master formulation, in the form of a cream), achieving high cure rates in different types of viral warts (particularly in warts caused by HPVs), and with a minimum incidence of adverse effects associated with its use (the vast majority of the adverse effects are mild and temporary). The main limitation preventing its use from being widespread is the cost of said treatment and the shortage of this substance in some countries.

Therefore, given the high incidence of HPV infections, warts and, particularly, HPV mucocutaneous infections, the variability of treatments that are not guaranteed to be effective, the possible discomforts caused (pain, itch, superinfections, etc.) both while the lesion is present and after applying some treatments, the deterioration of the quality of life due to functional and occupational disabilities, as well as treatment costs and medical leaves, there is still a need for an alternative treatment against HPV infections which is capable of eliminating mucocutaneous lesions with minimal effects on normal tissues, thereby causing as little discomfort as possible for patients.

### BRIEF DESCRIPTION OF THE INVENTION

A **first aspect** of the invention relates to the use of a pyrophosphate ion analogue in the production of a drug for the prevention, alleviation or treatment of an HPV infection-related disease. Alternatively, it relates to a pyrophosphate ion analogue for the use thereof in the prevention, alleviation or treatment of an HPV infection-related disease.

In a preferred embodiment of this aspect of the invention, the pyrophosphate ion analogue is phosphonoformic acid or foscarnet, or any of the salts thereof, preferably any pharmaceutically acceptable salt, esters, tautomers, polymorphs, hydrates that are pharmaceutically acceptable, or an isomer, prodrugs, derivatives, solvates or analogues, or any of the combinations thereof.

In another preferred embodiment of this aspect of the invention, the HPV infection-related disease is selected from the list consisting of: squamous cell carcinoma of the skin, cervical intraepithelial neoplasia, cervical cancer, anal intraepithelial neoplasia, cancer of the oral mucosa, tonsil cancer, pharyngeal cancer, laryngeal cancer, esophageal cancer, breast cancer, prostate cancer, ovarian cancer, vulvar cancer, urethral cancer, penile cancer and skin cancer, melanocytic tumors, common wart, palmer-plantar wart, periungual wart, flat skin wart, anogenital warts (condylomata), epidermodysplasia verruciformis, bowenoid papulosis, focal epithelial hyperplasia (oral), oral papillomas, or any of the combinations thereof. Alternatively, it relates to a pyrophosphate ion analogue, and more preferably foscarnet, for the use thereof in the treatment of a disease selected from the list consisting of: squamous cell carcinoma of the skin, cervical intraepithelial neoplasia, cervical cancer, anal intraepithelial neoplasia, cancer of the oral mucosa, tonsil cancer, pharyngeal cancer, laryngeal cancer, esophageal cancer, breast cancer, prostate cancer, ovarian cancer, vulvar cancer, urethral cancer, penile cancer and skin cancer, melanocytic tumors, common wart, palmer-plantar wart, periungual wart, flat skin wart, anogenital warts (condylomata), epidermodysplasia verruciformis, bowenoid papulosis, focal epithelial hyperplasia (oral), oral papillomas, or any of the combinations thereof

In another even more preferred embodiment, the HPV infection-related disease is a mucocutaneous infection.

In another preferred embodiment of this aspect of the invention, the HPV infection-related disease is a common wart, a palmer-plantar wart, a periungual wart, a flat skin wart or an anogenital wart.

In another preferred embodiment of this aspect of the invention, the route of administration is the topical route.

A **second aspect** of the invention relates to a composition, hereinafter composition of the invention, comprising a pyrophosphate ion analogue according to the first aspect of the invention.

The composition can further comprise a pharmaceutically acceptable vehicle, and it is more preferably a pharmaceutical composition. In another preferred embodiment, the pharmaceutical composition further comprises excipients.

In another preferred embodiment of this aspect of the invention, the composition further comprises another active ingredient. More preferably, the active ingredient is selected from acids such as salicylic acid, lactic acid, glutaraldehyde, a keratolytic agent, an immunostimulating agent, an immunomodulator, a cryotherapy agent, or any of the combinations thereof. In another more preferred embodiment, the additional active ingredient is selected from salicylic acid, tretinoin, silver nitrate, dinitrochlorobenzene, squaric acid, interferon, imiquimod, cantharidin, bleomycin, 5-fluorouracil, podophyllotoxin or any of the combinations thereof.

A **third aspect** of the invention relates to a dosage form, hereinafter dosage form of the invention, comprising a pyrophosphate ion analogue according to the first aspect of the invention, or the pharmaceutical composition of the invention.

In another preferred embodiment of the invention, the dosage form is selected from the list comprising: plaster, ointment, paste, cream, solution, suspension, emulsion, lotion, liniment, jelly, gel, foam, powder, or any of the combinations thereof. In another more preferred embodiment of this aspect of the invention, the dosage form is a gel. In another more preferred embodiment of this aspect of the invention, the dosage form is foam.

A **fourth aspect** of the invention relates to a combined preparation, hereinafter combined preparation of the invention, comprising:
- a component A comprising the composition according to any of claims 6-11, or a dosage form according to any of claims 12-15, and
- a component B comprising at least another active ingredient.

In a preferred embodiment of this aspect of the invention, the active ingredient of component B is selected from an acidic agent, glutaraldehyde, a keratolytic agent, an immunostimulating agent, an immunomodulator, a cryotherapy agent or any of the combinations thereof. In another preferred embodiment of this aspect of the invention, the active ingredient of component B is selected from salicylic acid, tretinoin, silver nitrate, dinitrochlorobenzene, squaric acid, interferon, imiquimod, cantharidin, bleomycin, 5-fluorouracil, podophyllotoxin or a combination thereof.

A **fifth aspect** of the invention relates to a patch or dressing, patch or dressing of the invention, comprising a composition of the invention, a dosage form of the invention, or a combined preparation of the invention.

In a preferred embodiment of this aspect of the invention, the patch is padded.

A **sixth aspect** of the invention relates to a kit of parts or combined preparation, hereinafter kit of parts or combined preparation of the invention, comprising two components:
- a component A comprising the composition of the invention, and
   b) a component B, comprising another active ingredient, and the active ingredient preferably being selected from an acidic agent (such as lactic acid or salicylic acid), glutaraldehyde, a keratolytic agent, an immunostimulating agent, an immunomodulator, a cryotherapy agent or any of the combinations thereof.

In another more preferred embodiment, component B is selected from salicylic acid, tretinoin, silver nitrate, dinitrochlorobenzene, squaric acid, interferon, imiquimod, cantharidin, bleomycin, 5-fluorouracil, podophyllotoxin or a combination thereof.

A **seventh aspect** of the invention relates to the use of a composition of the invention, a dosage form of the invention, a combined preparation of the invention, a patch or dressing of the invention, or a kit of parts of the invention, in the production of a drug for the prevention, alleviation or treatment of an HPV infection-related disease. Alternatively, it relates to the composition of the invention, the dosage form of the invention, the combined preparation of the invention, the patch or dressing of the invention, the kit of parts of the invention, for the use thereof for the prevention, alleviation or treatment of an HPV infection-related disease.

In a preferred embodiment of this aspect of the invention, the HPV infection-related disease is selected from the list consisting of: squamous cell carcinoma of the skin, cervical intraepithelial neoplasia, cervical cancer, anal intraepithelial neoplasia, cancer of the oral mucosa, tonsil cancer, pharyngeal cancer, laryngeal cancer, esophageal cancer, breast cancer, prostate cancer, ovarian cancer, vulvar cancer, urethral cancer, penile cancer and skin cancer, melanocytic tumors, common wart, palmer-plantar wart, periungual wart, flat skin wart, anogenital warts (condylomata), epidermodysplasia verruciformis, bowenoid papulosis, focal epithelial hyperplasia (oral), oral papillomas or any of the combinations thereof.

In another preferred embodiment of this aspect of the invention, the HPV infection-related disease is a mucocutaneous infection.

In another preferred embodiment of this aspect of the invention, the HPV infection-related disease is a common wart, a palmer-plantar wart, a periungual wart, a flat skin wart or an anogenital wart.

In another preferred embodiment of this aspect of the invention, the route of administration of the composition or the dosage form, a combined preparation, a patch or dressing, or a kit of parts, is the topical route.

### DESCRIPTION OF THE DRAWINGS

**Figure 1** shows the progression of the lesion on the patient of Example 2: at the beginning (A) and after treatment (B).
**Figure 2** shows the progression of the lesion on the patient of Example 3: at the beginning (A) and after treatment (B).

### DETAILED DESCRIPTION OF THE INVENTION

The antiviral effect of pyrophosphonate analogues, such as phosphonoformic acid (PFA) and its analogues and derivatives, inhibiting replications of various viruses, has been known for some time now (see McKenna's patent documents US5072032 and US5183812; D. W. Hutchinson, et al., Synthesis and Biochemical Properties of Some Pyrophosphate Analogues, Biophosphates and Their Analogues - Synthesis. Structure, Metabolism and Activity, K.S. Bruzik and W. J. Stec (Eds.), Elsevier Science Publishers, B. V., 1987, 441-450; and Helgstrands et al., Science, 201: 819-821 (1978)). Trisodium phosphonoformate (PFA, Foscarnet), a pyrophosphate analogue, has been described to inhibit HIV reverse transcriptase (HIV, RT) with an ID₅₀ of about 1 µm, and to also inhibit various DNA polymerases of the herpes virus, including the DNA polymerase of the CMV (see patent document US5072032). Patent documents 4386081 and 4591583 of Helgstrand *et al.* disclose phosphonoformic acid esters of alkyl, alkylene, alkoxy and related cyclic and aromatic groups, and some of said groups have been shown to inhibit the herpes virus and the intracellular functions and multiplication of the influenza virus. Patent document US5194654 of Hosteller et al. discloses phospholipid derivatives of phosphonoacids, their incorporation into liposomes and use as selective antiviral and antiretroviral agents.

However, despite the fact that these analogues have been known for some time now, the use of pyrophosphate ion analogues for the treatment of diseases caused by HPV infection has not been proposed.

The authors of the present invention have identified the therapeutic effect of the pyrophosphate ion analogue, and specifically foscarnet, against diseases caused by human papillomavirus infections, and particularly against mucocutaneous infections, where it constitutes an alternative to current treatments.

This treatment has the apparent advantages of being an effective, low-cost antiviral that is applied topically on mucocutaneous infections caused by the HPV and offers the possibility of an easy application only on the affected area, without significant side effects up until now. Furthermore, topical administration of said antiviral does not have the adverse effects typical of systemic administration.

Therefore, a **first aspect** of the invention relates to the use of a pyrophosphate ion analogue in the production of a drug for the prevention, alleviation or treatment of an HPV infection-related disease. Alternatively, it relates to a pyrophosphate ion analogue for the use thereof in the prevention, alleviation or treatment of an HPV infection-related disease.

In addition to phosphonoformate and phosphonoacetate, which are strong inhibitors, among other analogues, 2-phosphonopropionate, 2-phenylphosphonoacetate, oxalate, carbonylphosphate, methanolhydroxy-diphosphonate and hypophosphate also inhibit the DNA polymerase activity of HSV-1.

In a preferred embodiment of this aspect of the invention, the pyrophosphate ion analogue is phosphonoformic acid or foscarnet, or any of the salts thereof, preferably any pharmaceutically acceptable salt, esters, tautomers, polymorphs, hydrates that are pharmaceutically acceptable, or an isomer, prodrugs, derivatives, solvates or analogues, or any of the combinations thereof.

In another preferred embodiment of this aspect of the invention, the HPV infection-related disease is selected from the list consisting of: squamous cell carcinoma of the skin, cervical intraepithelial neoplasia, cervical cancer, anal intraepithelial neoplasia, cancer of the oral mucosa, tonsil cancer, pharyngeal cancer, laryngeal cancer, esophageal cancer, breast cancer, prostate cancer, ovarian cancer, vulvar cancer, urethral cancer, penile cancer and skin cancer, melanocytic tumors, common wart, palmer-plantar wart, periungual wart, flat skin wart, anogenital warts (condylomata), epidermodysplasia verruciformis, bowenoid papulosis, focal epithelial hyperplasia (oral), oral papillomas, or any of the combinations thereof.

In another even more preferred embodiment, the HPV infection-related disease is a mucocutaneous infection.

In another preferred embodiment of this aspect of the invention, the HPV infection-related disease is a common wart, a palmer-plantar wart, a periungual wart, a flat skin wart or an anogenital wart.

In another preferred embodiment of this aspect of the invention, the disease is related with a mucocutaneous infection, preferably molluscum contagiosum.

Molluscum contagiosum is a benign viral infection caused by a virus, member of the *Poxvirus family,* usually affecting children between 2 and 5 years of age with incidence rates between 5 and 8%. It also affects sexually active adults, and HIV-infected patients are particularly susceptible to this infection.

It is a common infection in children and occurs when a child comes into direct contact with a lesion. It is usually seen on the face, neck, armpits, arms and hands, although it can also surface in any part of the body, with the exception of the palms of the hands and the soles of the feet.

The virus can spread through contact with contaminated objects, such as towels, clothing or toys. The virus also propagates through sexual contact.

In another preferred embodiment of this aspect of the invention, the route of administration is the topical route.

In this specification, "phosphonoformic acid" or "foscarnet" is understood as the conjugated base of phosphonoformic acid HOOC-PO₃H₂, or a tautomer, an isomer, a pharmaceutically acceptable salt, a derivative or a prodrug thereof. It is an antiviral used for cytomegalovirus infections, particularly for retinitis caused by cytomegalovirus in people who have compromised immune systems and/or are ganciclovir-resistant. It has the following formula (I):

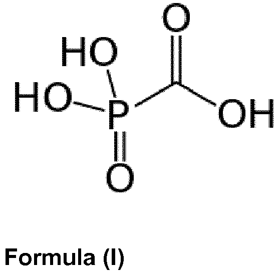

Its IUPAC nomenclature is phosphonoformic acid and its CAS number is 63585-09-1.

Pyrophosphate ion analogues, and specifically phosphonoformic acid (foscarnet), are known by the person skilled in the art, for example, but without limitation, sulfur-containing phosphonoformic acid derivatives obtained by substituting one or more of the five oxygen atoms of the original phosphonoformate molecule with a sulfur atom, such as for example, those described in European patent application EP99933950: Na3TPTFA4; Na3TPDTFA5, NaTPFA6, NaTPTNFA10; NaTPTNFA101, NaTPTNFA12. Patent documents 4386081 and 4591583 of Helgstrand et al. disclose phosphonoformic acid esters of alkyl, alkylene, alkoxy and related cyclic and aromatic groups. Patent document US5194654 of Hosteller et al. discloses phospholipid derivatives of phosphonoacids.

The compounds of the present invention represented by formula (I) can include isomers, depending on the presence of multiple bonds, including optical isomers or enantiomers, depending on the presence of chiral centers. Individual isomers, enantiomers or diastereoisomers and mixtures thereof fall within the scope of the present invention, i.e., the term isomer also refers to any mixture of isomers, such as diastereomers, racemates, etc., even the optically active isomers thereof or mixtures thereof in different proportions. Individual enantiomers or diastereoisomers, as well as the mixtures thereof, can be separated by means of conventional techniques.

Prodrugs of the compounds of formula (I) also fall within the scope of this invention. As it is used herein, the term "prodrug" includes any derivative of a compound of formula (I), for example and in a non-limiting manner: esters (including carboxylic acid esters, amino acid esters, phosphate esters, sulfonate esters of metal salts, etc.), carbamates, amides, etc., which, when administered to an individual, can be converted directly or indirectly into said compound of formula (I) in the mentioned individual. Advantageously, said derivative is a compound which increases the bioavailability of the compound of formula (I) when administered to an individual or enhances the release of the compound of formula (I) into a biological compartment. The nature of said derivative is not critical as long as it can be administered to an individual and provide the compound of formula (I) in a biological compartment of an individual. Said prodrug can be prepared by means of conventional methods known by the persons skilled in the art.

As it is used herein, the term "derivative" includes both pharmaceutically acceptable compounds, i.e., derivatives of the compound of formula (I) which can be used in the production of a drug or food compositions, and non-pharmaceutically acceptable derivatives, since they may be useful in the preparation of pharmaceutically acceptable derivatives.

The compounds of the invention can be in crystalline form as free compounds or solvates. In this sense, as it is used herein the term "solvate" includes both pharmaceutically acceptable solvates, i.e., solvates of the compound of formula (I) which can be used in the production of a drug, and non-pharmaceutically acceptable solvates, which may be useful in the preparation of pharmaceutically acceptable solvates or salts. The nature of the pharmaceutically acceptable solvate is not critical as long as it is pharmaceutically acceptable. In a particular embodiment, the solvate is a hydrate. Solvates can be obtained by means of conventional solvation methods known by the persons skilled in the art.

For application in therapy, the compounds of formula (I), their salts, prodrugs or solvates will preferably be in a pharmaceutically acceptable or substantially pure form, i.e., having a pharmaceutically acceptable level of purity, excluding normal pharmaceutical additives such as diluents and carriers, and not including materials considered as toxic at normal dosage levels. The levels of purity of the active ingredient are preferably greater than 50%, more preferably greater than 70%, and even more preferably greater than 90%. In a preferred embodiment, the levels of impurity of the compound of formula (I), or their salts, solvates or prodrugs are greater than 95%.

As it is used herein, the term "drug" refers to any substance used for the prevention, diagnosis, alleviation, treatment or cure of diseases in humans and animals. In the context of the present invention, the disease is a proliferative disease, preferably a proliferative disease of the skin or mucosae, even more preferably a human papillomavirus infection, and still more preferably a wart.

### PHARMACEUTICAL COMPOSITION, DOSAGE FORM OF THE INVENTION

A **second aspect** of the invention relates to a composition, hereinafter composition of the invention, comprising a pyrophosphate ion analogue according to the first aspect of the invention.

The compositions of the present invention can be formulated for administration to an animal, and more preferably a mammal, including a human, in many forms known in the state of the art. In this sense, the compositions can be, without limitation, in sterile aqueous solution or biological fluids, such as serum. The aqueous solutions may or may not be buffered and they have additional active or inactive components. The additional components include salts for modulating ionic force, preservatives and other substances including, but not limited to, antimicrobial agents, antioxidants, chelating agents, and the like, and nutrients including glucose, dextrose, vitamins and minerals. Alternatively, the compositions can be prepared for administration in solid form. The compositions can be combined with various inert excipients or vehicles including, but not limited to, binders such as microcrystalline cellulose, gum tragacanth or gelatin; excipients such as starch or lactose; dispersing agents such as alginic acid or cornstarch; lubricants such as magnesium stearate; glidants such as colloidal silicon dioxide; sweetening agents such as sucrose or saccharin; or flavoring agents such as mint or methyl salicylate.

The composition can further comprise a pharmaceutically acceptable vehicle, and it is more preferably a pharmaceutical composition. In another preferred embodiment, the pharmaceutical composition further comprises excipients.

In another preferred embodiment of this aspect of the invention, the composition further comprises another active ingredient. More preferably, the active ingredient is selected from acids such as salicylic acid, lactic acid, glutaraldehyde, a keratolytic agent, an immunostimulating agent, an immunomodulator, a cryotherapy agent, or any of the combinations thereof. In another more preferred embodiment, the additional active ingredient is selected from salicylic acid, tretinoin, silver nitrate, dinitrochlorobenzene, squaric acid, interferon, imiquimod, cantharidin, bleomycin, 5-fluorouracil, podophyllotoxin or any of the combinations thereof.

As it is used herein, the term "active ingredient", "active substance", "pharmaceutically active substance", or "pharmaceutically active ingredient" means any component that may provide pharmacological activity or another different effect in the diagnosis, cure, mitigation, treatment, or prevention of a disease, or that affects the body structure or function of humans or other animals. The term includes those components that promote a chemical change in the production of the drug and are present in the drug in an envisaged modified form, providing the specific activity or effect.

A **third aspect** of the invention relates to a dosage form, hereinafter dosage form of the invention, comprising a pyrophosphate ion analogue according to the first aspect of the invention, or the pharmaceutical composition of the invention.

In another preferred embodiment of the invention, the dosage form is selected from the list comprising: plaster, ointment, paste, cream, solution, suspension, emulsion, lotion, liniment, jelly, gel, foam, powder, or any of the combinations thereof. In another more preferred embodiment of this aspect of the invention, the dosage form is a gel. In another more preferred embodiment of this aspect of the invention, the dosage form is foam.

In this specification, "dosage form" is understood to be the mixture of one or more active ingredients, with or without additives, having physical characteristics for suitable dosing, preservation, administration and bioavailability.

A "plaster" or "patch" is a dosage form consisting of a solid or semisolid form which contains active ingredient/ingredients and additive/additives extended on a piece of fabric, plastic or adhesive strip, serving as a support and protection, in addition to furnishing an occlusive and macerating action that further allows direct contact with the skin and softens with body temperature.

A "salve" or "ointment" is a dosage form consisting of a preparation having a soft consistency which contains active ingredient/ingredients and additive/additives incorporated to a suitable base that imparts mass and consistency thereto. It is applied on the skin and mucosae and adheres to same. This base can be fat- or water-soluble, generally anhydrous or with a maximum percentage of water. It is also referred to as a hydrophilic salve when it contains a base that can be washed away or removed with water.

A "paste" is a dosage form consisting of a semisolid form which contains active ingredient/ingredients and additive/additives, prepared based on a high concentration of insoluble powders (20% to 50%), in fatty or aqueous bases, absorbents or weak abrasives combined with soaps.

A "cream" is a dosage form consisting of a liquid or semisolid preparation which contains active ingredient/ingredients and additive/additives required for obtaining an emulsion, generally an oil-in-water emulsion, with a water content greater than percent.

A "solution" is a dosage form consisting of a transparent and homogeneous liquid preparation that is obtained by dissolving active ingredient/ingredients and additive/additives in water and intended for external or internal use. Injectable solutions as well as solutions intended for the eyes and ears must be sterile solutions.

A "suspension" is a dosage form consisting of a dispersed system, made up of two phases which contain active ingredient/ingredients and additive/additives. One of the phases, the continuous or external phase is generally a liquid or semisolid and the dispersed or internal phase is made up of solids (active ingredients) that are insoluble but dispersible in the external phase. Injectable suspensions must be sterile.

An "emulsion" is a dosage form consisting of a heterogeneous system, generally made up of two liquids not miscible with one another, in which the dispersed phase is made up of small globules distributed in a vehicle in which they are not miscible. The dispersed phase is also known as internal phase and the dispersion means is known as external or continuous phase. Emulsions include water-in-oil or oil-in-water emulsions and they can be presented as semisolids or liquids. The active ingredient/ingredients and additive/additives can be in the external or internal phase.

A "lotion" is a dosage form which can be presented as a solution, suspension or emulsion that contains active ingredient/ingredients and additive/additives, and the dispersing agent of which is mainly water.

A "liniment" is a dosage form consisting of a presentation in the form of a liquid, solution or emulsion containing active ingredient/ingredients and additive/additives, with an aqueous, alcoholic or fatty vehicle.

A "jelly" is a dosage form consisting of a semisolid colloid which contains active ingredient/ingredients and additive/additives, the water-soluble base of which generally consists of gums such as gum tragacanth; other bases used are: glycerin, pectin, alginates, boroglycerine compounds, synthetic derivatives or natural substances such as carboxymethyl cellulose.

A "gel" is a dosage form consisting of a semisolid preparation which contains active ingredient/ingredients and additive/additives, solids in a liquid which can be water, alcohol or oil, such that a network of particles trapped in the liquid phase is formed.

"Foam" is a dosage form consisting of a semisolid preparation made up of two phases: a liquid phase incorporating the active ingredient/ingredients and additive/additives, and another gas phase incorporating the propelling gas so that the product comes out in the form of a mist.

"Powder" is a dosage form consisting of a solid form which contains active ingredient/ingredients and additive/additives that are finely ground and mixed to assure homogeneity.

The compositions and/or the formulations thereof can be administered to an animal, including a mammal and, therefore to a human, in many ways, including, but not limited to, intraperitoneally, intravenously, intramuscularly, subcutaneously, intracecally, intraventricularly, orally, enterally, parenterally, intranasally or topically.

Preferably, the compositions of the invention are administered topically. The dosage for obtaining a therapeutically effective amount depends on many factors, such as, the age, weight, sex and tolerance of the mammal, for example. In the sense used in this description, the expression "therapeutically effective amount" refers to the amount (or concentration) of the pyrophosphate ion analogue which causes the desired effect and will be determined in general, among other causes, by the characteristics typical of said prodrugs, derivatives or analogues and the therapeutic effect to be achieved. "Adjuvants" and "pharmaceutically acceptable vehicles" which can be used in said compositions are vehicles known by the persons skilled in the art.

A **fourth aspect** of the invention relates to a combined preparation, hereinafter combined preparation of the invention, comprising:
- a component A comprising the composition according to any of claims 6-11, or a dosage form according to any of claims 12-15, and
- a component B comprising at least another active ingredient.

In a preferred embodiment of this aspect of the invention, the active ingredient of component B is selected from an acidic agent, glutaraldehyde, a keratolytic agent, an immunostimulating agent, an immunomodulator, a cryotherapy agent or any of the combinations thereof. In another preferred embodiment of this aspect of the invention, the active ingredient of component B is selected from salicylic acid, tretinoin, silver nitrate, dinitrochlorobenzene, squaric acid, interferon, imiquimod, cantharidin, bleomycin, 5-fluorouracil, podophyllotoxin or a combination thereof.

### PATCH OF THE INVENTION

A **fifth aspect** of the invention relates to a patch or dressing, patch or dressing of the invention, comprising a composition of the invention, a dosage form of the invention, or a combined preparation of the invention.

In a preferred embodiment of this aspect of the invention, the patch is padded.

### KITS AND COMBINED PREPARATION OF THE INVENTION

A **sixth aspect** of the invention relates to a kit of parts or combined preparation, hereinafter kit of parts or combined preparation of the invention, comprising two components:
- a component A comprising the composition of the invention, and
   b) a component B, comprising another active ingredient, and the active ingredient preferably being selected from an acidic agent (such as lactic acid or salicylic acid), glutaraldehyde, a keratolytic agent, an immunostimulating agent, an immunomodulator, a cryotherapy agent or any of the combinations thereof.

It must be emphasized that in this specification, the term "combined preparation" or also referred to "juxtaposition" means that the components of the combined preparation need not be present as a union, for example in a real composition, to be available for combined, separate or sequential application. The expression "juxtaposed" therefore means that it is not necessarily a real combination in view of the physical separation of the components. The components of the combined preparation can be administered separately, simultaneously or sequentially.

In a preferred embodiment of this aspect of the invention, the kit further comprises additionally:
c) a dressing.

In another preferred embodiment of this aspect of the invention, elements (b) and/or (c) of the kit of the invention are integrated in the patch or dressing of the invention.

In another more preferred embodiment, component B is selected from salicylic acid, tretinoin, silver nitrate, dinitrochlorobenzene, squaric acid, interferon, imiquimod, cantharidin, bleomycin, 5-fluorouracil, podophyllotoxin or a combination thereof.

A **seventh aspect** of the invention relates to the use of a composition of the invention, a dosage form of the invention, a combined preparation of the invention, a patch or dressing of the invention, or a kit of parts of the invention, in the production of a drug for the prevention, alleviation or treatment of an HPV infection-related disease.

In a preferred embodiment of this aspect of the invention, the HPV infection-related disease is selected from the list consisting of: squamous cell carcinoma of the skin, cervical intraepithelial neoplasia, cervical cancer, anal intraepithelial neoplasia, cancer of the oral mucosa, tonsil cancer, pharyngeal cancer, laryngeal cancer, esophageal cancer, breast cancer, prostate cancer, ovarian cancer, vulvar cancer, urethral cancer, penile cancer and skin cancer, melanocytic tumors, common wart, palmer-plantar wart, periungual wart, flat skin wart, anogenital warts (condylomata), epidermodysplasia verruciformis, bowenoid papulosis, focal epithelial hyperplasia (oral), oral papillomas or any of the combinations thereof.

In another preferred embodiment of this aspect of the invention, the HPV infection-related disease is a mucocutaneous infection.

In another preferred embodiment of this aspect of the invention, the HPV infection-related disease is a common wart, a palmer-plantar wart, a periungual wart, a flat skin wart or an anogenital wart.

In another preferred embodiment of this aspect of the invention, the route of administration of the composition or the dosage form, a combined preparation, a patch or dressing, or a kit of parts, is the topical route.

Throughout the description and claims, the word "comprises" and variants thereof do not seek to exclude other features, techniques, additives, components or steps. For the persons skilled in the art, other objects, advantages and features of the invention will be inferred in part from the description and in part from the practice of the invention. The following examples and drawings are provided by way of illustration and do not seek to limit the present invention.

### EXAMPLES

### Example 1. Preparation of a foscarnet composition for topical application

### FORCARNET GEL FOR TOPICAL USE

The active ingredient is dissolved at 2% in a hypromellose gel buffered to pH 7.4 with phosphate buffer, and parabens are added to prevent microbial contaminations.

| | |
|---|---|
| Foscarnet | 2% |
| Methyl parahydroxybenzoate | 0.08% |
| Propyl parahydroxybenzoate | 0.02% |
| Sodium lauryl sulfate | 4% |

Hypromellose in the amount required to achieve the desired consistency.

Phosphate buffer to maintain a pH of 7.4.

The preparation is kept cold (4°C) once prepared and it has a theoretical stability of 6 months.

### Example 2. Trials in patients

### Example 2.1

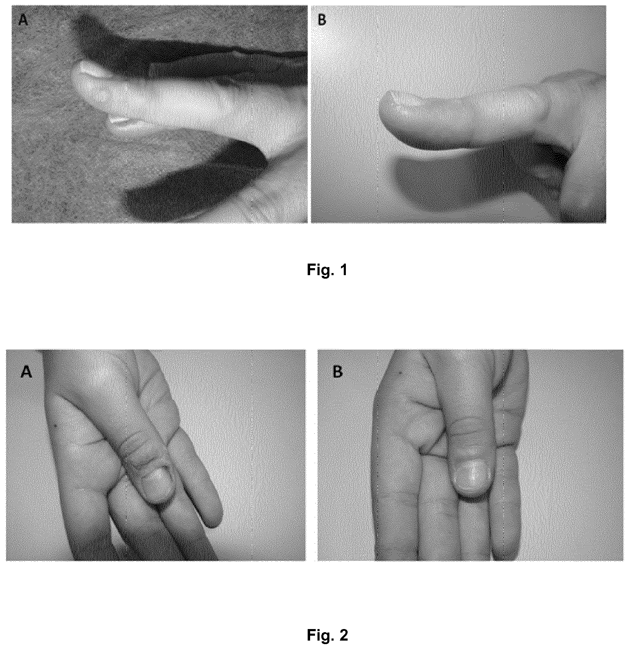

Female patient, 53 years of age, who had a lesion on the pad of the third finger of her right hand since 2012 and was unresponsive to prior cryotherapy, electro-curettage, salicylic acid and imiquimod treatments. The patient started on 2% topical foscarnet gel on 08-01-2014, 2 times a day, and the lesion went away after 6 weeks (see Figure 1)

### Example 2.2

Female child patient, 9 years of age, who had a keratotic periungual wart lesion on the thumb of her right hand since January 2012 and had received prior cryotherapy, imiquimod, Veregen cream and oral zinc treatments without any results. The patient started on 2% topical foscarnet gel in September 2013 with 1 application at night and the lesion went away in 3 weeks without any side effects (see Figure 2).

### Example 3 Expanding trials in patients

A total of 28 patients, from which 64.3% are female patients and 35.7% male patients to whom treatment was administered, were studied. The mean age was 26 years old (6-64) and with a median age of 16 years. Warts on the hands (fingers or palms) were the most commonly observed, occurring in 67.9% of the cases, followed by periungual warts, occurring in 32.1% of the cases.

It is important to point out that 96.4% of the patients had received prior treatments without any results, where the most common treatments are cryotherapy involving 78.6% of said patients and topical keratolytic drugs involving 67.9% of them.

Most of the patients (71.4%) applied the treatment 2 times a day and in 78.6% of the cases, the area of application was banded for the night. The rest of the patients applied the treatment 1 or 3 times/day.

The mean duration of the treatment was 11.57 (3-28) weeks.

The percentage of response was 71.4% (39.3% full response and 32.1% partial response).

The response was obtained at a mean of 5.25 (± 4.98) weeks.

Only 10.7% of said patients suffered from very mild localized adverse effects (erythema or slight irritation/itch).

A series of tables with analysis data are included below. The results show that foscarnet is useful in the production of drugs for the prevention, alleviation or treatment of an HPV infection-related disease where, in addition to being an alternative, it is a good option in the cases in which the patients had received prior treatments without any results.

### Table 1. Table indicating the frequency according to location

**Table 1A. Hand involvement**

| **Hand involvement** | | | | | |
|---|---|---|---|---|---|
| | | Frequency | Percentage | Percentage valid | Percentage Accumulated |
| Valid | No | 9 | 32.1 | 32.1 | 32.1 |
| | Yes | 19 | 67.9 | 67.9 | 100.0 |
| | Total | 28 | 100.0 | 100.0 | |

**Table 1B. Leg involvement**

| **Leg involvement** | | | | | |
|---|---|---|---|---|---|
| | | Frequency | Percentage | Percentage valid | Percentage accumulated |
| Valid | No | 20 | 71.4 | 71.4 | 71.4 |
| | Yes | 8 | 28.6 | 28.6 | 100.0 |
| | Total | 28 | 100.0 | 100.0 | |

**Table 1C. Periungual involvement**

| **Periungual involvement** | | | | | |
|---|---|---|---|---|---|
| | | Frequency | Percentage | Percentage valid | Percentage accumulated |
| Valid | No | 19 | 67.9 | 67.9 | 67.9 |
| | Yes | 9 | 32.1 | 32.1 | 100.0 |
| | Total | 28 | 100.0 | 100.0 | |

**Table 1D. Genital involvement**

| **Genital involvement** | | | | | |
|---|---|---|---|---|---|
| | | Frequency | Percentage | Percentage valid | Percentage accumulated |
| Valid | No | 28 | 100.0 | 100.0 | 100.0 |

**Table 1E. Face involvement**

| **Face involvement** | | | | | |
|---|---|---|---|---|---|
| | | Frequency | Percentage | Percentage valid | Percentage accumulated |
| Valid | No | 22 | 78.6 | 78.6 | 78.6 |
| | Yes | 6 | 21.4 | 21.4 | 100.0 |
| | Total | 28 | 100.0 | 100.0 | |

**Table 1F. Various locations**

| **Various locations** | | | | | |
|---|---|---|---|---|---|
| | | Frequency | Percentage | Percentage valid | Percentage accumulated |
| Valid | No | 15 | 53.6 | 55.6 | 55.6 |
| | Yes | 12 | 42.9 | 44.4 | 100.0 |
| | Total | 27 | 96.4 | 100.0 | |
| System losses | | 1 | 3.6 | | |
| Total | | 28 | 100.0 | | |

**Table 2. Distribution by sexes**

| **Sex of the patient** | | | | | |
|---|---|---|---|---|---|
| | | Frequency | Percentage | Percentage valid | Percentage accumulated |
| Valid | Male | 10 | 35.7 | 35.7 | 35.7 |
| | Female | 18 | 64.3 | 64.3 | 100.0 |
| | Total | 28 | 100.0 | 100.0 | |

**Table 3. Previous treatments**

| **Previous treatments** | | | | | |
|---|---|---|---|---|---|
| | | Frequency | Percentage | Percentage valid | Percentage accumulated |
| Valid | No | 1 | 3.6 | 3.6 | 3.6 |
| | Yes | 27 | 96.4 | 96.4 | 100.0 |
| | Total | 28 | 100.0 | 100.0 | |

**Table 3A. Previous cryotherapy treatment**

| **Cryotherapy** | | | | | |
|---|---|---|---|---|---|
| | | Frequency | Percentage | Percentage valid | Percentage accumulated |
| Valid | No | 6 | 21.4 | 21.4 | 21.4 |
| | Yes | 22 | 78.6 | 78.6 | 100.0 |
| | Total | 28 | 100.0 | 100.0 | |

**Table 3B. Previous treatment with keratolytic agents**

| **Keratolytic agents** | | | | | |
|---|---|---|---|---|---|
| | | Frequency | Percentage | Percentage valid | Percentage accumulated |
| Valid | No | 9 | 32.1 | 32.1 | 32.1 |
| | Yes | 19 | 67.9 | 67.9 | 100.0 |
| | Total | 28 | 100.0 | 100.0 | |

**Table 3C. Previous treatment with imiquimid**

| **Imiquimod** | | | | | |
|---|---|---|---|---|---|
| | | Frequency | Percentage | Percentage valid | Percentage Accumulated |
| Valid | No | 24 | 85.7 | 85.7 | 85.7 |
| | Yes | 4 | 14.3 | 14.3 | 100.0 |
| | Total | 28 | 100.0 | 100.0 | |

**Table 3D. Previous treatment with electrocurettage**

| **Electrocurettage** | | | | | |
|---|---|---|---|---|---|
| | | Frequency | Percentage | Percentage valid | Percentage Accumulated |
| Valid | No | 20 | 71.4 | 71.4 | 71.4 |
| | Yes | 8 | 28.6 | 28.6 | 100.0 |
| | Total | 28 | 100.0 | 100.0 | |

**Table 3E. Previous treatment with cidofovir**

| **Cidofovir** | | | | | |
|---|---|---|---|---|---|
| | | Frequency | Percentage | Percentage valid | Percentage Accumulated |
| Valid | No | 21 | 75.0 | 75.0 | 75.0 |
| | Yes | 7 | 25.0 | 25.0 | 100.0 |
| | Total | 28 | 100.0 | 100.0 | |

**Table 3F. Previous treatment with bleomycin**

| **Bleomycin** | | | | | |
|---|---|---|---|---|---|
| | | Frequency | Percentage | Percentage valid | Percentage Accumulated |
| Valid | No | 27 | 96.4 | 96.4 | 96.4 |
| | Yes | 1 | 3.6 | 3.6 | 100.0 |
| | Total | 28 | 100.0 | 100.0 | |

**Table 3G. Other treatments**

| **Other treatments** | | | | |
|---|---|---|---|---|
| | Frequency | Percentage | Percentage valid | Percentage Accumulated |
| Valid | 21 | 75.0 | 75.0 | 75.0 |
| Curettage and TCA | 1 | 3.6 | 3.6 | 78.6 |
| Retirides | 1 | 3.6 | 3.6 | 82.1 |
| Retirides and TCA | 1 | 3.6 | 3.6 | 85.7 |
| Retirides, Zinnox ioox | 1 | 3.6 | 3.6 | 89.3 |
| Intralesional TV vaccine, several PDTs | 1 | 3.6 | 3.6 | 92.9 |
| Veregen | 1 | 3.6 | 3.6 | 96.4 |
| VTV | 1 | 3.6 | 3.6 | 100.0 |
| Total | 28 | 100.0 | 100.0 | |

**Table 4. Number of applications of foscarnet treatment per day**

| **No. of applications/day** | | | | | |
|---|---|---|---|---|---|
| | | Frequency | Percentage | Percentage valid | Percentage Accumulated |
| Valid | One | 5 | 17.9 | 17.9 | 17.9 |
| | Two | 20 | 71.4 | 71.4 | 89.3 |
| | Three | 3 | 10.7 | 10.7 | 100.0 |
| | Total | 28 | 100.0 | 100.0 | |

**Table 5. Application of treatment with the area subsequently banded**

| **Application with banding** | | | | | |
|---|---|---|---|---|---|
| | | Frequency | Percentage | Percentage valid | Percentage Accumulated |
| Valid | Zero | 6 | 21.4 | 21.4 | 21.4 |
| | One | 22 | 78.6 | 78.6 | 100.0 |
| | Total | 28 | 100.0 | 100.0 | |

**Table 6. Response to treatment**

| **Response to treatment** | | | | | |
|---|---|---|---|---|---|
| | | Frequency | Percentage | Percentage valid | Percentage Accumulated |
| Valid | No | 8 | 28.6 | 28.6 | 28.6 |
| | Partial | 9 | 32.1 | 32.1 | 60.7 |
| | Complete | 11 | 39.3 | 39.3 | 100.0 |
| | Total | 28 | 100.0 | 100.0 | |

**Table 7. Adverse effects to treatment**

| **Adverse effects to treatment** | | | | | |
|---|---|---|---|---|---|
| | | Frequency | Percentage | Percentage valid | Percentage Accumulated |
| Valid | No | 25 | 89.3 | 89.3 | 89.3 |
| | Yes | 3 | 10.7 | 10.7 | 100.0 |
| | Total | 28 | 100.0 | 100.0 | |

**Table 8. Recurrence**

| **Recurrence** | | | | | |
|---|---|---|---|---|---|
| | | Frequency | Percentage | Percentage valid | Percentage Accumulated |
| Valid | No | 27 | 96.4 | 100.0 | 100.0 |
| Lost | System | 1 | 3.6 | | |
| | Total | 28 | 100.0 | | |

**Table 9. Statistics**

| | | Age of starting foscarnet | Duration of treatment (weeks) | Response to treatment |
|---|---|---|---|---|
| N | Valid | 28 | 28 | 28 |
| | Lost | 0 | 0 | 0 |
| Mean | | 26.00 | 11.57 | |
| Median | | 16.00 | 12.00 | |
| Std. Dev. | | 20.861 | 6.221 | |
| Minimum | | 6 | 3 | |
| Maximum | | 64 | 28 | |

**Tables 9B, 9C, 9D and 9E. Tables of frequencies**

| **Age of starting foscarnet** | | | | | |
|---|---|---|---|---|---|
| | | Frequency | Percentage | Percentage valid | Percentage Accumulated |
| Valid | 6 | 1 | 3.6 | 3.6 | 3.6 |
| | 7 | 3 | 10.7 | 10.7 | 14.3 |
| | 8 | 1 | 3.6 | 3.6 | 17.9 |
| | 9 | 3 | 10.7 | 10.7 | 28.6 |
| | 10 | 2 | 7.1 | 7.1 | 35.7 |
| | 11 | 2 | 7.1 | 7.1 | 42.9 |
| | 13 | 1 | 3.6 | 3.6 | 46.4 |
| | 15 | 1 | 3.6 | 3.6 | 50.0 |
| | 17 | 1 | 3.6 | 3.6 | 53.6 |
| | 18 | 1 | 3.6 | 3.6 | 57.1 |
| | 24 | 2 | 7.1 | 7.1 | 64.3 |
| | 25 | 1 | 3.6 | 3.6 | 67.9 |
| | 41 | 1 | 3.6 | 3.6 | 71.4 |
| | 47 | 1 | 3.6 | 3.6 | 75.0 |
| | 49 | 1 | 3.6 | 3.6 | 78.6 |
| | 52 | 1 | 3.6 | 3.6 | 82.1 |
| | 53 | 1 | 3.6 | 3.6 | 85.7 |
| | 57 | 1 | 3.6 | 3.6 | 89.3 |
| | 62 | 1 | 3.6 | 3.6 | 92.9 |
| | 63 | 1 | 3.6 | 3.6 | 96.4 |
| | 64 | 1 | 3.6 | 3.6 | 100.0 |
| | Total | 28 | 100.0 | 100. | |

**Table 9C.**

| **Duration of treatment (weeks)** | | | | | |
|---|---|---|---|---|---|
| | | Frequency | Percentage | Percentage valid | Percentage Accumulated |
| Valid | 3 | 2 | 7.1 | 7.1 | 7.1 |
| | 4 | 5 | 17.9 | 17.9 | 25.0 |
| | 5 | 1 | 3.6 | 3.6 | 28.6 |
| | 8 | 1 | 3.6 | 3.6 | 32.1 |
| | 9 | 2 | 7.1 | 7.1 | 39.3 |
| | 10 | 1 | 3.6 | 3.6 | 42.9 |
| | 12 | 3 | 10.7 | 10.7 | 53.6 |
| | 13 | 1 | 3.6 | 3.6 | 57.1 |
| | 16 | 10 | 35.7 | 35.7 | 92.9 |
| | 20 | 1 | 3.6 | 3.6 | 96.4 |
| | 28 | 1 | 3.6 | 3.6 | 100.0 |
| | Total | 28 | 100.0 | 100.0 | |

**Table 9D**

| **Response to treatment** | | | | | |
|---|---|---|---|---|---|
| | | Frequency | Percentage | Percentage valid | Percentage Accumulated |
| Valid | No | 8 | 28.6 | 28.6 | 28.6 |
| | Partial | 9 | 32.1 | 32.1 | 60.7 |
| | Complete | 11 | 39.3 | 39.3 | 100.0 |
| | Total | 28 | 100.0 | 100.0 | |

**Table 9E**

| **Statistics** | | |
|---|---|---|
| Time effect starts (weeks) | | |
| N | Valid | 28 |
| | Lost | 0 |
| Mean | | 5.25 |
| Median | | 4.00 |
| Std. Dev. | | 4.979 |
| Minimum | | 0 |
| Maximum | | 16 |

## Claims

1. Use of a pyrophosphate ion analogue in the production of a drug for the prevention, alleviation or treatment of an HPV infection-related disease.

2. Use of a pyrophosphate ion analogue according to the preceding claim, wherein the pyrophosphate ion analogue is phosphonoformic acid or foscarnet.

3. Use of a pyrophosphate ion analogue according to any of claims 1-2, wherein the HPV infection-related disease is selected from the list consisting of: squamous cell carcinoma of the skin, cervical intraepithelial neoplasia, cervical cancer, anal intraepithelial neoplasia, cancer of the oral mucosa, tonsil cancer, pharyngeal cancer, laryngeal cancer, esophageal cancer, breast cancer, prostate cancer, ovarian cancer, vulvar cancer, urethral cancer, penile cancer and skin cancer, melanocytic tumors, common wart, palmer-plantar wart, periungual wart, flat skin wart, anogenital warts (condylomata), epidermodysplasia verruciformis, bowenoid papulosis, focal epithelial hyperplasia (oral), oral papillomas, or any of the combinations thereof.

4. Use of a pyrophosphate ion analogue according to any of claims 1-3, wherein the HPV infection-related disease is a mucocutaneous infection.

5. Use of a pyrophosphate ion analogue according to any of claims 1-4, wherein the HPV infection-related disease is a common wart, a palmer-plantar wart, a periungual wart, a flat skin wart or an anogenital wart.

6. Use of a pyrophosphate ion analogue according to any of claims 1-5, wherein the route of administration of the drug is the topical route.

7. A composition comprising a pyrophosphate ion analogue as described in any of claims 1-6.

8. The composition according to the preceding claim, wherein the composition further comprises another active ingredient.

9. The composition according to any of claims 7-8, wherein the active ingredient is selected from an acidic agent, glutaraldehyde, a keratolytic agent, an immunostimulating agent, an immunomodulator, a cryotherapy agent or any of the combinations thereof.

10. The composition according to any of claims 7-9, wherein the active ingredient is selected from salicylic acid, tretinoin, silver nitrate, dinitrochlorobenzene, squaric acid, interferon, imiquimod, cantharidin, bleomycin, 5-fluorouracil, podophyllotoxin, or any of the combinations thereof.

11. The composition according to any of claims 7-10, wherein the composition is a pharmaceutical composition.

12. A dosage form comprising a pyrophosphate ion analogue according to any of claims 1-6, or the pharmaceutical composition according to any of claims 7-11.

13. The dosage form according to the preceding claim, wherein the dosage form is selected from the list comprising: plaster, ointment, paste, cream, solution, suspension, emulsion, lotion, liniment, jelly, gel, foam, powder, or any of the combinations thereof.

14. The dosage form according to any of claims 12-13, wherein the dosage form is a gel.

15. The dosage form according to any of claims 12-14, wherein the dosage form is foam.

16. A combined preparation comprising:
• a component A comprising the composition according to any of claims 6-11, or a dosage form according to any of claims 12-15, and
• a component B comprising at least another active ingredient.

17. The combined preparation according to the preceding claim, wherein the active ingredient of component B is selected from an acidic agent, glutaraldehyde, a keratolytic agent, an immunostimulating agent, an immunomodulator, a cryotherapy agent or any of the combinations thereof.

18. The combined preparation according to any of claims 16-17, wherein the active ingredient of component B is selected from salicylic acid, tretinoin, silver nitrate, dinitrochlorobenzene, squaric acid, interferon, imiquimod, cantharidin, bleomycin, 5-fluorouracil, podophyllotoxin or a combination thereof.

19. A patch or dressing comprising the composition according to any of claims 6-10, a dosage form according to any of claims 12-15, or a combined preparation according to any of claims 16-18.

20. The patch or dressing according to the preceding claim, wherein the patch is padded.

21. A kit of parts comprising component A as described in any of claims 16-18, and additionally,
a) component B as described in any of claims 16-18, and/or
b) the patch or dressing according to any of claims 19-20.

22. Use of a composition according to any of claims 6-10, a dosage form according to any of claims 12-15, a combined preparation according to any of claims 16-18, a patch or dressing according to any of claims 19-20, or a kit of parts according to claim 21, in the production of a drug for the prevention, alleviation or treatment of an HPV infection-related disease.

23. Use of a composition, a dosage form, a combined preparation, a patch or dressing, or a kit of parts according to the preceding claim, wherein the HPV infection-related disease is selected from the list consisting of: squamous cell carcinoma of the skin, cervical intraepithelial neoplasia, cervical cancer, anal intraepithelial neoplasia, cancer of the oral mucosa, tonsil cancer, pharyngeal cancer, laryngeal cancer, esophageal cancer, breast cancer, prostate cancer, ovarian cancer, vulvar cancer, urethral cancer, penile cancer and skin cancer, melanocytic tumors, common wart, palmer-plantar wart, periungual wart, flat skin wart, anogenital warts (condylomata), epidermodysplasia verruciformis, bowenoid papulosis, focal epithelial hyperplasia (oral), oral papillomas or any of the combinations thereof.

24. Use of a composition, a dosage form, a combined preparation, a patch or dressing, or a kit of parts according to any of claims 22-23, wherein the HPV infection-related disease is a mucocutaneous infection.

25. Use of a composition, a dosage form, a combined preparation, a patch or dressing, or a kit of parts according to any of claims 22-24, wherein the HPV infection-related disease is a common wart, a palmer-plantar wart, a periungual wart, a flat skin wart or an anogenital wart.

26. Use of a composition, a dosage form, a combined preparation, a patch or dressing, or a kit of parts according to any of claims 22-25, wherein the route of administration of the composition or the dosage form, a combined preparation, a patch or dressing, or a kit of parts is the topical route.
